# EUROPÄISCHE PATENTSCHRIFT

(11) **EP 0 527 298 B1**
(45) Veröffentlichungstag und Bekanntmachung des Hinweises auf die Patenterteilung: **30.07.1997**
(21) Anmeldenummer: 92108192.3
(22) Anmeldetag: 15.05.1992
(51) Int. Cl.: C07D 513/04, A61K 31/54

(54) **Verfahren zur Herstellung von Pipazetat**
Process for the preparation of pipazethate
Procédé de préparation de pipazethate

(30) Priorität: 14.08.1991 DE 4126863
(43) Veröffentlichungstag der Anmeldung: 17.02.1993
(73) Patentinhaber: ASTA Medica Aktiengesellschaft, D-01277 Dresden (DE)
(72) Erfinder: Lehmann, Bernd, Dr., W-7750 Konstanz 19 (DE); Petrat, Bernhard, W-7750 Konstanz (DE)

(56) Entgegenhaltungen:
- FR-A- 2 326 194
- JUSTUS LIEBIGS ANNALEN DER CHEMIE. Bd. 673, 1964, WEINHEIM DE Seiten 102 - 112 W. A. SCHULER ET AL. 'Synthesen von 4-Aza-phenothiazinen, II. Derivate der 4- Aza-phenothiazin-10-carbonsäure'

## Beschreibung

Die vorliegende Erfindung betrifft ein verbessertes Verfahren zur Herstellung von Pipazetat, insbesondere dessen Hydrochlorid (Selvigon®, (INN), D-254).

Die Synthese dieses pharmazeutischen Wirkstoffes wird in den Patentschriften DBP 1012919 und DBP 1055538 sowie Liebigs Ann. 673, 102-12 (1964) beschrieben: 1-Azaphenothiazin wird in Chlorbenzol bei 170°C Badtemperatur mit Phosgen behandelt. Hierbei bildet sich in 60,9 % Ausbeute 1-Azaphenothiazincarbonylchlorid, welches mit einem Überschuß von bis zwei Äquivalenten Piperidinoethoxyethanol zu Pipazetat umgesetzt wird. Die Ausbeute an Pipazetat-Base, deren Reinheit nicht weiter spezifiziert ist, beträgt bezogen auf 1-Azaphenothiazin 44,3 % der Theorie und bezogen auf Piperidinoethoxyethanol 29,0 % der Theorie. Die Ausbeute an Pipazetathydrochlorid wird nicht angegeben. Erfahrungsgemäß muß hier jedoch mit einem Verlust von mindestens 15 % der Theorie gerechnet werden, so daß sich die Ausbeuten auf schätzungsweise 38 % der Theorie bezogen auf 1-Azaphenothiazin und 25 % der Theorie bezogen auf Piperidinoethoxyethanol reduzieren. Diese Ausbeuten sind sehr unbefriedigend. Auch Optimierung während der jahrzehntelangen Produktion von Pipazetat hat an den ursprünglichen Ausbeuten nichts geändert.

Im langjährigen Durchschnitt wurde bezogen auf eingesetztes 1-Azaphenothiazin 39,2 % Ausbeute und 18,2 % Ausbeute bezogen auf eingesetztes Piperidinoethoxyethanol erzielt.

Durch zusätzliche aufwendige Verfahrensschritte konnte nicht umgesetztes 1-Azaphenothiazin und Piperidinoethoxyethanol teilweise zurückgewonnen werden. Hierdurch stieg die Umsatz-Ausbeute jedoch nur auf 43,9 % bezogen auf 1-Azaphenothiazin bzw. 33,6 % bezogen auf Piperidinoethoxyehtanol.

Das bisherige Herstellungsverfahrne liefert keine guten Ausbeuten und ist auch umweltbelastend.

Aufgabe der Erfindung ist es, das bekannte Verfahren zu verbessern und zu vereinfachen.

Es wurde nun überraschend gefunden, daß 1-Azaphenoothiazin in Gegenwart von Pyridin oder Pyridinderivaten bereits bei Raumtemperatur quantitativ ohne Bildung kernchlorierter 1-Azaphenothiazinderivate zu 1-Azaphenothiazin-10-carbonylchlorid umgesetzt wird.

Diese niedrige Reaktionstemperatur ermöglicht jetzt auch den Einsatz unproblematischer Lösungsmittel wie zum Beispiel Toluol. Chlorbenzol, das bislang verwendet wird, führt als chlorierter Kohlenwasserstoff zur erhöhten AOX-Werten im Abwasser.

Das erhaltende 1-Azaphenothiazin-10-carbonylchlorid läßt sich beispielsweise entgegen der aus Liegigs Ann, 673, 102-112 (1964) bekannten Herstellung in Gegenwart von Pyridin bereits mit 1 Mol Piperidinoethoxyethanol/Mol Säurechlorid zu Pipazetathydrochlorid umsetzen. Bislang wurden für diesen Reaktionsschritt mindestens 2 Mol Piperidinoethoxyethanol benötigt.

Gemäß der bekannten Herstellung (Liebigs Ann, 673, 102-112 (1964)) werden 2,5 Mol Piperidinoethoxyethanol/Mol Säurechlorid verwendet.
Dieser Einsatz überschüssigen Piperidinoethoxyethanols, der unvollständige Umsatz des 1-Azaphenothiazins, die starke Bildung von kernchlorierten Nebenprodukten, die Verwendung von Chlorbenzol bei den Reaktionen und der notwendige Wechsel zu Toluol im weiteren Verlauf des Verfahrens führt zu einem sehr aufwendigen und auch umständlichen vielstufigen Prozeß.

Die Erfindung betrifft ein verbessertes Verfahren zur Darstellung von Pipazetathydrochlorid. Es besteht darin, 1-Azaphenothiazin zweckmäßig in einem inerten Lösungsmittel wie zum Beispiel aliphatischen Kohlenwasserstoffen, die flüssig sind und die aus 6 bis 10 Kohlenwasserstoffatomen bestehen oder aromatischen Kohlenwasserstoffen, die flüssig sind und aus 7 bis 8 Kohlenstoffatomen bestehen, zum Beispiel Hexan, Heptan und Octan oder Toluol, Xylole in Gegenwart von Pyridin oder Pyridinderivaten umzusetzen.

Die Menge dieses Amins bezogen auf 1 Mol 1-Azaphenothiazin beträgt im allgemeinen mit 1 bis 3 Mol, insbesondere 1,05-1,3 Mol.

Die Menge an Phosgen, bezogen auf 1 Mol 1-Azaphenothiazin beträgt im allgemeinen 1 bis 3 Mol, vorzugsweise 1 bis 2 Mol, insbesondere 1,3-1,5 Mol. Das Phosgen kann gasförmig, flüssig oder als Lösung eingebracht werden.

Die Reaktionstemperatur der Umsetzung des 1-Azaphenothiazins mit den Phosgen liegt bei 40-60°C.

Die Reaktionszeit beträgt zum Beispiel 1 bis 2 Stunden, beispielsweise 1,5 Stunden.

Als Pyridinderivate kommen zum Beispiel infrage: Chinolin, Isochinolin sowie die höheren Homologe des Pyridins, Chinolins und Isochinolins, beispielsweise alkylsubstituierte Pyridine, Chinoline und Isochinoline. Als Alkylsubstituent kommt vorzugsweise die Methylgruppe infrage. Beispiele für solche methylsubstituierten Pyridine sind:
α-Picolin, β-Picolin, γ-Picolin, Lutidine wie 2,4-Dimethylpyridin, 2,5-Dimethylpyridin, 2,3-Dimethylpyridin, 2,6-Dimethylpyridin, Collidine wie 2,4,6-Trimethylpyridin, 2,3,4-Trimethylpyridin, 2,3,5-Trimethylpyridin, 2,3,6-Trimethylpyridin. Vorzugsweise wird Pyridin verwendet. Im Zuge der Reaktion bilden sich die entsprechenden Hydrochloride.

Gegebenenfalls vorhandenes überschüssiges Phosgen wird entfernt (zum Beispiel mittels eines Inertgasstroms oder Vakuums) und anschließend bei 20-70^{o}C, vorzugsweise 40-60°C werden 0,95-1,3 Mol, vorzugsweise 1-1,05 Mol Piperidinoethoxyethanol und 0-0,2 Mol einer basischen Substanz zugesetzt.
Als basische Substanzen für diesen 2. Schritt kommen die bereits erwähnten Pyridine beziehungsweise Pyridinderivate infrage; es können hier jedoch auch andere basische Substanzen verwendet werden wie zum Beispiel tertiäre aliphatische Amine mit niederen Alkylresten (C₁ - C₆), beispielsweise Triethylamin. Das erhaltende Gemisch aus Pipazetathydrochlorid und Hydrochlorid der jeweils zugesetzten Base kann vom Lösungsmittel abgetrennt und direkt aus aliphatischen Alkoholen mit 2 bis 5 Kohlenstoffatomen, die geradkettig oder verzweigt sein können und die eine oder mehrere Hydroxylgruppen tragen, zu reinem Pipazetathydrochlorid umkristallisiert werden. Besonders günstig ist die folgende wässrige Aufarbeitung, die sich auch für die Herstellung anderer Salze empfiehlt.
Das Reaktionsgemisch aus Pipazetathydrochlorid und dem Hydrochlorid der eingesetzten Base wird hierzu mit Wasser versetzt. Bezogen auf 1 Gewichtsteil 1-Azaphenothiazin als Ausgangssubstanz werden 2 bis 5 Gewichtsteile Wasser verwendet.
Die wässrige Phase wird abgetrennt und eventuell mit 0,04 Gewichtsteilen Aktivkohle geklärt. Diese hochkonzentrierte wässrige Lösung von Pipazetathydrochlorid und Pyridinhydrochlorid hat eine pH-Wert von ca. 4 und stellt eine stabile Transportform für den Wirkstoff dar.

Zur Gewinnung von Pipazetat wird sie mit einem mit Wasser nicht mischbaren Lösungsmittel, wie zum Beispiel Toluol, Xylol und der wässrigen Lösung einer Base wie zum Beispiel Natronlauge (zum Beispiel 5-52 Gewichts%), Kalilauge (zum Beispiel 5-53 Gewichts%), Natriumcarbonat (zum Beispiel 10-18 Gewichts% oder auch als Feststoff) oder Kaliumcarbonat (zum Beispiel 10-52 Gewichts% oder auch als Feststoff) bis zu einem Wert von pH 7-9, vorzugsweise 8,1 versetzt.
Andere basische Substanzen wie zum Beispiel CaO, MgO, Mg(OH)₂, Ca(OH)₂ sind ebenfalls einsetzbar.
Besonders bevorzugt ist hierbei wässrige Natronlauge.

Die organische Phase wird dann im Vakuum eingeengt und liefert Pipazetat-Base in einer Ausbeute von beispielsweise 95% der Theorie. Dies entspricht gegenüber die Literatur einer Ausbeutesteigerung von 114%. Die Pipazetat-Base wird dann in einem aliphatischem C₂-C₆-Alkohol (geradkettig oder verzweigt), der auch 2, 3 oder mehr Hydroxylgruppen enthalten kann wie zum Beispiel Isopropanol, Ethanol, Butanol, gelöst, und durch Zusatz von Säure auf leicht sauren pH-Wert von beispielsweise 5,5-6 gebracht. Als Säuren kommen hierfür insbesondere solche Säuren infrage, deren Anionen physiologisch unbedenklich sind und üblicherweise für die Salzbildung von Arzneimittelwirkstoffen verwendet werden. Vorzugsweise verwendet man die entsprechenden anorganischen Säuren, vorzugsweise in wässriger oder alkoholischer Lösung, insbesondere wässrige oder alkoholische Salzsäure beziehungsweise Chlorwasserstoff.

Zum Beispiel verwendet man alkoholische (Alkohole mit 1 - 6 C-Atomen, insbesondere 2 - 4 C-Atomen) Lösungen solcher Säuren, beispielsweise isopropanolische HCl vom Gehalt von 5 bis 35 %. Das so erhaltene Kristallisat wird abzentrifugiert und gegebenenfalls aus aliphatischen Alkoholen mit 2 bis 5 Kohlenstoffatomen, die geradkettig oder verzweigt sein können und die eine oder mehrere Hydroxylgruppen tragen, umkristallisiert.
Man erhält auf diese Weise beispielsweise Salze des Pipazetats wie zum Beispiel hochreine Pipazetathydrochlorid in hoher Ausbeute bezogen auf 1-Azaphenothiazin bzw. Piperidinoethoxyethanol.

### Beispiel 1

200,3 g (1,0 Mol) 1-Azaphenothiazin und 1,1 l Toluol werden azeotrop entwässert. Nach dem Abkühlen auf 30°C werden 88,8 g (1,1 Mol) Pyridin zugesetzt.
Innerhalb von 1,2 Stunden leitet man 131 g (1,32 Mol) Phosgen in den Reaktionskolben ein, wobei die Reaktionstemperatur auf 55°C steigt und durch Kühlung zwischen 55°C und 58°C gehalten wird. Bei 55°C läßt man noch 1,5 Stunden nachrühren.
Man beginnt nun überschüssiges Phosgen mittels Stickstoff bei 50-60°C auszutreiben.

Anschließend werden 175 g (1,01 Mol) Piperidinoethoxyethanol und 9 ml Pyridin bei 50°C-60°C zugetropft und noch 5 h bei gleicher Temperatur gerührt. Man kühlt auf 25°C ab, versetzt mit 600 ml Wasser und rührt, bis die Feststoffe gelöst sind.
Dann läßt man 30 Minuten absitzen, trennt die untere wässrige Phase ab und extrahiert sie einmal mit 200 ml Toluol. Die wässrige Phase wird mit 8 g Aktivkohle (Chemviron) geklärt. Die Lösung wird mit 830 ml Toluol versetzt und unter Rühren mit 25%iger Natronlauge auf pH 8,1 gestellt. Hierfür werden ca. 300 g (1,88 Mol) benötigt.
Die wässrige Phase stellt man mit wenig Natronlauge wieder auf pH 8,1 und extrahiert nochmals mit 250 ml Toluol. Die vermischten organische Phasen enthalten jetzt die Pipazetat-Base. Sie werden bei 50°C im Vakuum eingeengt. Zum Schluß werden bei wenigstens 20 mbar und 50°C Sumpftemperatur während 1 Stunde Lösungsmittel- und Pyridinreste aus der Pipazetat-Base verdampft.

Man erhält 380 g (95% d. Th.) an Pipazetat-Base.
Sie wird bei 50°C in 390 ml Isopropanol gelöst und mit isopropanolischer Salzsäure auf pH 5,8 gestellt.
Man kühlt auf 0°C und filtriert nach 12 h das Pipazetathydrochlorid ab. Nach dem Trocknen bei 50°C im Vakuum erhält man 375 g (86% d. Th.) an Pipazetathydrochlorid.

Die Umkristallisation aus 530 ml Isopropanol liefert 363 g (83% d. Th.) an reinem Pipazetathydrochlorid.

## Patentansprüche

1. Verfahren zur Herstellung von Pipazetat aus 1-Azaphenothiazin und Phosgen und anschließender Reaktlon des erhaltenen 1-Azaphenothiazin-10-carbonsäurechlorids mit 2-(2-Piperidinoethoxy)ethanol, dadurch gekennzeichnet, daß die Umsetzung des 1-Azaphenothiazins mit Phosgen in Gegenwart von Pyridin oder Pyridinderivaten erfolgt und die Temperatur bei der Umsetzung zwischen 40°C und 60°C liegt.

2. Verfahren nach Anspruch 1, dadurch gekennreichnet, daß 2-(2-Piperidinoethoxy)-ethanol in Mengen von 0,95 - 1,3 Mol pro Mol 1-Azaphenothiazin eingesetzt wird.

3. Verfahren zur Isolierung von reinem Pipazetathydrochlorid, dadurch gekennzeichnet, daß die gemäß Anspruch 1 erhaltene Reaktionsmischung mit einer Base versetzt wird, das Pipazetat mit einem inerten Lösungsmittel extrahiert und das Pipazetathydrochlorid mittels Chlorwasserstoff oder wässriger beziehungsweise alkoholischer HCl ausgefällt und isoliert wird.

## Claims

1. Method for the preparation of pipazethate from 1-azaphenothiazine and phosgene and subsequent reaction of the resulting 1-azaphenothiazine-10-carboxylic acid chloride with 2-(2-piperidinoethoxy)ethanol, characterised in that the reaction of 1-azaphenothiazine with phosgene takes place in the presence of pyridine or pyridine derivatives and the temperature during the reaction is between 40°C and 60°C.

2. Method according to claim 1, characterised in that 2-(2-piperidinoethoxy)ethanol is used in quantities of from 0.95 to 1.3 mol per mol of 1-azaphenothiazine.

3. Method for the isolation of pure pipazethate hydrochloride, characterised in that a base is added to the reaction mixture obtained according to claim 1, the pipazethate is extracted with an inert solvent and the pipazethate hydrochloride is precipitated out by means of hydrogen chloride or aqueous or alcoholic HCl and isolated.

## Revendications

1. Procédé de production de pipazétate à partir de 1-azaphénothiazine et de phosgène et par réaction consécutive du chlorure de 1-azaphénothiazinyl-10-carboxylique avec le 2-(2-piperidinoéthoxyéthanol,
caractérisé en ce que
la réaction de la 1-azaphénothiazine avec le phosgène s'effectue en présence de pyridine ou de dérivés de la pyridine et en ce que la température au cours de la réaction se situe entre 40°C et 60°C.

2. Procédé selon la revendication 1,
caractérisé en ce que
l'on met en oeuvre le 2-(2-piperidinoéthoxyéthanol) en quantités allant de 0,95-1,3 mol par mole de 1-azaphénothiazine.

3. Procédé d'isolement du chlorhydrate de pipazétate pur,
caractérisé en ce que
le mélange réactionnel obtenu conformément à la revendication 1 est additionné d'une base, en ce que l'on extrait le pipazétate avec un solvant inerte et en ce que l'on précipite et isole le chlorhydrate de pipazétate à l'aide de gaz chlorhydrique ou de ClH aqueux ou alcoolique.
